**Europäisches Patentamt**

(19) **European Patent Office**

**Office européen des brevets**

(11) Publication number : **0 464 119 B1**

## (12) EUROPEAN PATENT SPECIFICATION

(45) Date of publication of patent specification :
18.10.95 Bulletin 95/42

(51) Int. Cl.⁶ : **G01N 21/75, G01N 33/53**

(21) Application number : **90905567.5**

(22) Date of filing : **21.03.90**

(86) International application number :
**PCT/GB90/00432**

(87) International publication number :
**WO 90/11510 04.10.90 Gazette 90/23**

(54) **ASSAY METHOD USING SURFACE PLASMON RESONANCE SPECTROMETRY.**

(30) Priority : **23.03.89 GB 8906781**

(43) Date of publication of application :
**08.01.92 Bulletin 92/02**

(45) Publication of the grant of the patent :
**18.10.95 Bulletin 95/42**

(84) Designated Contracting States :
**AT BE CH DE DK ES FR GB IT LI LU NL SE**

(56) References cited :
**EP-A- 257 955**
**EP-A- 276 142**
**EP-A- 286 195**
**GB-A- 2 173 895**

(73) Proprietor : **AMERSHAM INTERNATIONAL plc**
**Amersham Place**
**Little Chalfont Buckinghamshire HP7 9NA**
**(GB)**

(72) Inventor : **GARLAND, Peter, Bryan Hope**
**Cottage Sunnyway**
**Old Bosham**
**West Sussex PO18 8HQ (GB)**
Inventor : **POLLARD-KNIGHT, Denise, Vera 4**
**Drury Road**
**Harrow**
**Middlesex HA1 4BY (GB)**
Inventor : **CHARLES, Stephen, Alexander 5**
**Elio Close**
**Heydon Hill**
**Aylesbury**
**Bucks. HP1 9JB (GB)**

(74) Representative : **Pennant, Pyers et al**
**Stevens, Hewlett & Perkins**
**1 Serjeants' Inn**
**Fleet Street**
**London EC4Y 1LL (GB)**

## Description

This invention concerns methods of performing enzyme assays using the technique of surface plasmon resonance spectrometry (SPRS).

The phenomenon of SPR is well known and will not be described in detail (see EPA 305109 for example). Briefly, the intensity of monochromatic plane-polarised light (conveniently obtained from a laser) reflected from the interface between an optically transparent material, e.g. glass, and a thin metal film depends on the refractive index of material on the downstream side of the metal. Accordingly, by measuring changes in intensity of reflected light an indication can be obtained of changes in refractive index of material at a particular point on the downstream surface of the metal. The intensity of reflected light also varies with the angle of incidence, and reflectivity drops sharply to a minimum at a particular angle which is characteristic of the equipment.

EPA 276142 describes an assay method for an analyte using a SPRS surface on which a specific binding partner of the analyte is immobilised. Any analyte present in a sample forms a complex with its binding partner and is thus detected by SPRS.

In conventional assays, it is well known that an enzyme can be used to improve sensitivity by catalysing a reaction which converts substrates into large numbers of molecules of observable product. This invention assays enzymes or enzyme substrates by causing the product of the reaction to deposit a higher or lower refractive index substance on a SPRS detection surface.

The invention provides a surface plasmon resonance spectrometry assay method involving the use of a solid surface provided by a metallic layer applied to a block of material transparent to electromagnetic radiation, said solid surface carrying thereon, either an immobilised enzyme which catalyses a reaction between two or more reactants resulting in the production of a reaction product, or a substrate for the enzyme, which method comprises bringing into contact with the immobilised enzyme or enzyme substrate a fluid medium containing the remaining reactants and if not already present the enzyme,

characterised in that the reaction is performed to cause the reaction product to be deposited on the solid surface, deposition of the reaction product on the surface of the metallic layer being assayed by surface plasmon resonance spectrometry (SPRS).

The analyte may be an enzyme or an enzyme substrate. The SPRS equipment may be conventional. An enzyme may be immobilised on the surface of the metal e.g. silver layer by conventional means, e.g. covalent bonding or simply physical adsorption. A biotinylated enzyme may be immobilised on a metal surface to which streptavidin has been bound. Indeed immobilisation may involve any binding pair, including DNA/DNA or antibody/antigen. For example, a surface bound antibody can be used to capture an antigen which in turn captures an enzyme-antibody conjugate. Or an enzyme substrate may be immobilised on the solid surface of the metal layer.

It is necessary that the reaction product resulting from the action of the enzyme be immobilised on or exceedingly close (i.e. within a thin layer of a few hundred nanometers or less) to the metal surface. The reaction product may have a refractive index which is higher or lower than the material present in that layer. Examples of suitable enzymes are:-

a) Peroxidase, with $H_2O_2$ and diamino-benzidine (DAB) as substrates. The latter is converted to an insoluble product.

b) Certain oxidoreductases with NAD(P)H and a tetrazolium salt as substrate. The latter is converted to an insoluble product (a formazan). Other reductant and dye combinations exist. (See F P Altmann, 1972, "An introduction to the use of tetrazolium salts in quantitative enzyme cytochemistry" published by Koch-Light Laboratories, Colnbrook, Bucks, England).

c) Certain catalase enzymes are known to generate gas, bubbles of which can be retained close to the metal surface.

d) Other enzymes and their associated substrates are described in Aamlid et al, Chemistry and Industry, 20th February 1989, p106-8.

The assay performed by the method may be qualitative, i.e. simply to detect the presence or absence of an analyte in a sample, or quantitative. For quantitative assays, measurements may be made of the rate of change of reflectivity, and/or of the absolute reflectivity at a given time. Contact between the fluid medium and the immobilised enzyme may be static, but is more preferably dynamic e.g. by the fluid medium being caused to flow across the metal surface.

In one embodiment, the analyte to be assayed is a substrate of the immobilised enzyme, i.e. one of the reactants whose reaction is catalysed by the enzyme. In this case, the assay simply involves incorporating in the fluid medium an assay sample possibly comprising the analyte, together with any other reactants required by the enzyme.

In another embodiment, the analyte is an enzyme present, for example, as part of the electron transport chain of a bacterial cell. Viable bacteria deposit reaction product, whereas non-viable bacteria do not.

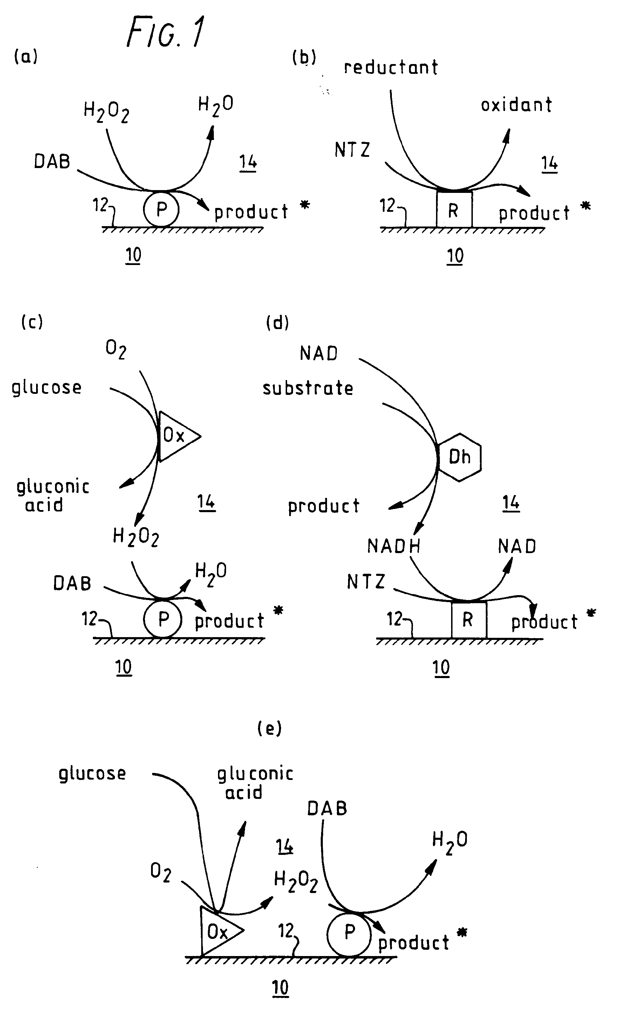

In yet another embodiment, use is made of a second enzyme, a product of which is a substrate for the immobilised enzyme. The system can be used to assay for a substrate for the second enzyme, thus greatly widening its applicability. The second enzyme may either be present in solution in the fluid medium, or may alternatively be immobilised on the solid surface. Reference is directed to the accompanying drawings in which Figure 1 comprises five diagrams (a), (b), (c), (d) and (e) showing reaction schemes of five embodiments of the invention. In each diagram, a metallic layer 10 forming part of an SPR system has a solid surface 12, in contact with which is a fluid medium 14. In diagram (a), a peroxidase enzyme P attached to the solid surface 12 can convert diamino-benzidine (DAB) into a surface associated insoluble product, in the presence of $H_2O_2$. This arrangement can therefore be used to assay $H_2O_2$.

Similarly, in diagram (b), an oxido-reductase enzyme R catalyses the reduction of a neotetrazolium dye (NTZ) to an insoluble product. This arrangement can therefore be used to assay the reductant.

Diagram (c) shows how an $H_2O_2$ generating enzyme such as glucose oxidase (Ox) can be functionally applied to peroxidase via $H_2O_2$ which is common to both reactions - a product of the former and a substrate for the latter. Thus the overall reaction in diagram (c) is:

glucose + $O_2$ + DAB -----> gluconic acid + $H_2O$ + insoluble product.

The system can thus be used to assay for glucose.

In diagram (d) a dehydrogenase enzyme (Dh) is functionally coupled to an oxido-reductase enzyme (R) via NADH which is common to both reactions - a product of the former and a substrate for the latter. When ethanol is the substrate, and acetaldehyde the product of the dehydrogenase enzyme, the overall reaction in diagram (d) is:

ethanol + NTZ ---> acetaldehyde + insoluble product

The system can be used in an assay for ethanol.

Diagram (e) corresponds to (c), except that the oxido reductase enzyme is shown immobilised on the solid surface 12 of the metal layer 10. This does not change the overall reaction occurring. A corresponding diagram could have been drawn, related to (d) and showing the dehydrogenase enzyme immobilised on the solid surface.

The following Examples illustrate the invention.

## Example 1

### Glucose Assay

### Slide Coating

10mM sodium phosphate pH 7.4 was pumped across a silver coated slide at 2µl/sec. This was followed by 0.5µM streptavidin in phosphate buffer at 2µl/sec and three washes of 0.3ml phosphate buffer. 0.25µM biotinylated-horseradish peroxidase in phosphate buffer was then flowed at 2µl/sec over the streptavidin coated slide. This slide was washed three times with 0.3ml of phosphate buffer at 2µl/sec. Reaction mixtures at different glucose concentrations prepared as described below were then flowed across the horseradish peroxidase coated silver slide and the change in reflectivity with time (rate) or the total change in reflectivity (equilibrium) monitored.

### Glucose Mix

The glucose reaction mix contained 10µl of glucose oxidase (10mg/ml), 10µl of glucose (stock 50mM) diluted to an appropriate concentration (0-20µM) in 1ml of sodium phosphate buffer pH 7.4. The mixture was incubated at room temperature for 10 minutes. lml of a diamino-benzidine solution (0.5mg/ml in sodium phosphate buffer pH 7.2) was then added and the mix was flowed over the coated slide as indicated above. A control of zero glucose was also performed. The results for equilibrium and rate measurements (slope) are shown in the following tables. A dose response for glucose concentration is observed.

| Glucose concentration (µM) | Final Change in Reflectivity (%) |
|---|---|
| 0 | 0 |
| 2.5 | 11 |
| 5 | 18 |
| 10 | 22 |
| 20 | 28 |

| Glucose concentration (µM) | Rate % Change/second |
|---|---|
| 0 | 0 |
| 2.5 | 0.023 |
| 5 | 0.0363 |
| 10 | 0.0488 |
| 20 | 0.0594 |

Example 2

Assay for Cholesterol

1 ml of 10 mM sodium phosphate buffer pH 7.4 (buffer 1) was pumped across the silver slide at 2 µl/sec. 0.5 µM streptavidin diluted in buffer 1 was then pumped across the silver at 2 µl/sec followed by three washes with 0.3 mls of buffer 1. A solution of biotinylated-horseradish peroxidase diluted to 100 nM in buffer 1 was then flowed across the silver surface at 2 µl/sec followed by three washes with 0.3 mls of buffer 1. The cholesterol reaction mix, which contained cholesterol oxidase (1 mg/ml), cholesterol (0-20 µM), diaminobenzedine (0.5 mg/ml) diluted in buffer 1 was then pumped across the silver surface containing the immobilised peroxidase at 2 µl/sec and the change in the SPR angle (reflectivity) monitored. As shown in the Table there is a change in the rate of the increase of reflectivity and final change in reflected light with the cholesterol concentration.

| Cholesterol concentration | Change in reflectivity |
|---|---|
| 0 | 0 |
| 10 µM | 24% |
| 20 µM | 45% |

**Claims**

1. A surface plasmon resonance spectrometry assay method involving the use of a solid surface provided by a metallic layer applied to a block of material transparent to electromagnetic radiation, said solid surface carrying thereon, either an immobilised enzyme which catalyses a reaction between two or more reactants resulting in the production of a reaction product, or a substrate for the enzyme, which method comprises bringing into contact with the immobilised enzyme or enzyme substrate a fluid medium containing the remaining reactants and if not already present the enzyme,

characterised in that the reaction is performed to cause the reaction product to be deposited on the solid surface, deposition of the reaction product on the surface of the metallic layer being assayed by surface plasmon resonance spectrometry.

2. A method as claimed in claim 1, wherein the solid surface carries thereon an immobilised enzyme.

3. A method as claimed in claim 2, wherein one of the said reactants is assayed by incorporating in the fluid medium an assay sample comprising the reactant to be assayed.

4. A method as claimed in claim 2, wherein there is also used a second enzyme a product of which is a substrate for the immobilised enzyme, and the method is used to assay for a substrate for the second enzyme.

5. A method as claimed in claim 4, wherein the second enzyme is present in the fluid medium.

6. A method as claimed in claim 4, wherein the second enzyme is immobilised on the solid surface.

## Patentansprüche

1. Oberflächenplasmonresonanzspektrometrie-Analyseverfahren, das die Verwendung einer festen Oberfläche einschließt, die von einer Metallschicht zur Verfügung gestellt wird, die auf einem Block aus für elektromagnetische Strahlung durchlässigem Material aufgetragen ist, wobei die feste Oberfläche darauf entweder ein immobilisiertes Enzym, das eine Reaktion zwischen zwei oder mehr Reaktanten katalysiert, die zur Produktion eines Reaktionsprodukts führt, oder ein Substrat für das Enzym trägt, wobei dieses Verfahren das In-Kontakt-bringen eines flüssigen Mediums, das die restlichen Reaktanten und, falls nicht schon vorhanden, das Enzym enthält, mit dem immobilisierten Enzym oder Enzymsubstrat umfaßt, dadurch gekennzeichnet, daß die Reaktion ausgeführt wird, um das Reaktionsprodukt zu veranlassen, auf der festen Oberfläche abgeschieden zu werden, wobei die Abscheidung des Reaktionsprodukts auf der Oberfläche der Metallschicht durch Oberflächenplasmonresonanzspektrometrie untersucht wird.

2. Verfahren gemäß Anspruch 1, worin die feste Oberfläche darauf ein immobilisiertes Enzym trägt.

3. Verfahren nach Anspruch 2, worin einer der Reaktanten durch Einbringung einer Analysenprobe, die den zu bestimmenden Reaktanten umfaßt, in das flüssige Medium bestimmt wird.

4. Verfahren nach Anspruch 2, worin auch ein zweites Enzym verwendet wird, von dem ein Produkt ein Substrat für das immobilisierte Enzym ist und das Verfahren verwendet wird, um nach einem Substrat für das zweite Enzym zu suchen.

5. Verfahren nach Anspruch 4, worin das zweite Enzym in dem flüssigen Medium anwesend ist.

6. Verfahren nach Anspruch 4, worin das zweite Enzym auf der festen Oberfläche immobilisiert ist.

## Revendications

1. Procédé de titrage par spectrométrie par résonance de plasmone en surface comprenant l'emploi d'une surface solide munie d'une couche métallique appliquée à un bloc de matériau transparent au rayonnement électromagnétique, ladite surface solide portant soit une enzyme immobilisée qui catalyse une réaction entre deux ou plusieurs réactifs résultant en la production d'un produit de réaction, soit un substrat pour l'enzyme, ce procédé comprenant la mise en contact avec l'enzyme immobilisée ou le substrat enzymatique d'un milieu fluide contenant les réactifs restants et l'enzyme si elle n'est pas déjà présente, caractérisé en ce que la réaction est conduite pour provoquer le dépôt du produit de réaction sur la surface solide, le dépôt du produit de réaction à la surface de la couche métallique étant titré par spectrométrie par résonance de plasmone en surface.

2. Procédé selon la revendication 1, dans lequel la surface solide porte une enzyme immobilisée.

3. Procédé selon la revendication 2, dans lequel l'un desdits réactifs est titré en incorporant dans le milieu fluide un échantillon de titrage comprenant le réactif à titrer.

4. Procédé selon la revendication 2, dans lequel on utilise également une seconde enzyme dont le produit est un substrat pour l'enzyme immobilisée et on utilise le procédé pour titrer comme substrat pour la seconde enzyme.

5. Procédé selon la revendication 4, dans lequel la seconde enzyme est présente dans le milieu fluide.

6. Procédé selon la revendication 4, dans lequel la seconde enzyme est immobilisée sur la surface solide.

# FIG. 1

(a)

(b)

(c)

(d)

(e)